# EUROPEAN PATENT APPLICATION

(11) **EP 4 516 202 A1**
(43) Date of publication of application: **05.03.2025**
(21) Application number: 24197522.6
(22) Date of filing: 30.08.2024
(51) Int. Cl.: A61B 1/00, A61B 1/005, A61B 1/008

(54) **METHOD OF ASSEMBLING ARTICULATION SECTIONS OF A FLEXIBLE ENDOSCOPE AND DEVICE FOR PRACTICING THE METHOD**

(30) Priority: 31.08.2023 US 202363535712 P
(71) Applicant: Karl Storz SE & Co. KG, 78532 Tuttlingen (DE)
(72) Inventor: Marchessault, John, 78532 Tuttlingen (DE); Tremblay, Robert, 78532 Tuttlingen (DE)

(57) **Abstract**

A method includes providing a first articulation section with a connection edge with an inner rivet receiving hole and an opposite edge and a second articulation section having a connection edge with an outer rivet receiving hole and an opposite edge. The first articulation section is placed on a cam assembly loading pin with a cam surface. A rivet shaft of a rivet is placed into the inner rivet receiving hole with the head of the rivet located at an interior side of the inner ear facing the cam surface. The second articulation section is placed adjacent to the first articulation section with the outer rivet receiving hole aligned with the inner rivet receiving hole. The loading pin is rotated to press the cam surface against the rivet head to move the rivet shaft through the outer rivet receiving hole. The rivet is fixed to the second articulation section.

## Description

### TECHNICAL FIELD

The invention relates to an endoscope and, more particularly, to a flexible endoscope and to a method of joining articulation sections of a flexible endoscope.

### TECHNICAL BACKGROUND

The conventional articulation section systems for an endoscope shaft employ an assembly method centered around the use of rivets to create a flexible and maneuverable structure. Such articulation section systems include several articulation sedections interconnected through rivets, with each rivet featuring two heads. During assembly, the rivets are inserted into corresponding holes within the joining rings or ears from the inner side of the articulation sections, and subsequently, a second head is formed from the outer side.

A primary issue of such articulation section systems relates to the clearance between the rivet body and the two joining ring holes or ears, which adversely affects its torque resistance. Additionally, maintaining precise control over the dimensions of the rivet's cylindrical body during assembly may be difficult, leading to uncertainties in the clearance between the rivet head and the articulation sections.

One approach has been to weld the cylindrical body or rivet shaft of each rivet to the inner flat surface of one of the articulation sections, resulting in a single head located outside the articulation sections frame. However, the welding process presents difficulties as to maintaining the desired clearance due to the force required to press the rivet onto the ring's flat surface, which can inadvertently deform the ring and cause clearance issues.

US8579801 (B2) and US8834356 (B2) are said to present a method that can ensure better and consistent guaranteed clearance between the rivets and the section rings, thereby enhancing the overall performance and reliability of conventional endoscope shaft articulation sections. The method involves aligning fastener holes of two endoscope articulation section frame members with each other on a mandrel. The mandrel has a front end and a rear end. A fastener (rivet) is inserted into the fastener holes, wherein the fastener holes are aligned by moving the two endoscope articulation section frame members on the mandrel from the front end. A portion of the fastener is pushed by a linear cam feature on the mandrel to move a body of the fastener in the fastener holes of the endoscope deflection section frame members based on a linear movement of the two endoscope articulation section frame members with each other on the mandrel. The fastener is welded from an outside direction of the frame members, to an exterior side of one of the endoscope articulation section frame members to thereby pivotably attach the endoscope deflection section frame members to each other.

The linear cam method has the disadvantage that the assembler must move the articulation section frame members to be joined along with the rivets along the assembly mandrel. This requires a great deal of moving components that all need to be aligned while in motion.

### SUMMARY OF THE INVENTION

It is an object of the invention to provide a method for loading of the articulation hinge point (rivet) into the articulation section of a flexible endoscope that avoids problems presented by prior art methods.

It is an object of the invention to provide a method for loading of the articulation hinge point (rivet) into the articulation section of a flexible endoscope that avoids a need to align numerous moving components during assembly.

According to the invention a method is provided that comprises providing a first articulation section (also referred to as a next to be connected articulation section) comprising a first section connection edge with an inner ear comprising a recessed rivet receiving portion having an inner rivet receiving hole and having an opposite section edge. A second articulation section (also referred to as the further adjacent articulation section ) is provided that comprises a second section connection edge with an outer ear comprising an outer rivet receiving portion having an outer rivet receiving hole and having an opposite section edge. The first articulation section is placed on a cam assembly tooling mandrel and loading pin, the loading pin having a cam surface configured to contact with a head of a rivet. A rivet shaft of a rivet is inserted into the inner rivet receiving hole of the inner ear, wherein the head of the rivet is located at an interior side of the inner ear and facing the cam surface. The second articulation section is disposed adjacent to the first articulation section with the outer rivet receiving hole of the outer ear aligned with the inner rivet receiving hole of the inner ear. The loading pin is rotated to press the cam surface of the loading pin against the head of the rivet to move the rivet shaft partially through inner rivet receiving hole of the inner ear and into and partially through the outer rivet receiving hole of the outer ear. The rivet is subsequently fixedly connected to the second articulation section.

The rivet is advantageously fixedly connected to the second (further adjacent) articulation section by welding the rivet, particularly the rivet shaft, to the outer ear of the second articulation section.

The first section connection edge of the first (next to be connected) articulation section further comprises another inner ear comprising another recessed rivet receiving portion having an another inner rivet receiving hole. The second section connection edge of the second (further adjacent) articulation section further comprises another outer ear comprising an another outer rivet receiving portion having another outer rivet receiving hole. Another rivet is provided.

Another rivet shaft of the other rivet is inserted into the other inner rivet receiving hole of the other inner ear. Another head of the other rivet is located at an interior side of the inner ear and facing the cam surface, wherein the cam surface is configured to contact with the head of the rivet and also to contact a head of the other rivet. The rotation of the loading pin presses the cam surface of the loading pin against both the head of the rivet to move the rivet shaft into and partially through the outer rivet receiving hole of the outer ear and also the head of the other rivet to move the other rivet shaft into and partially through the other outer rivet receiving hole of the other outer ear.

The method may comprise:
providing a first articulation section having a section distal or proximal edge with an first inner ear comprising a recessed rivet receiving portion having an inner rivet receiving hole and an second inner ear comprising a recessed rivet receiving portion having an inner rivet receiving hole and with a proximal or distal edge with a first outer ear comprising an outer rivet receiving portion having an outer rivet receiving hole and second outer ear comprising an outer rivet receiving portion having an outer rivet receiving hole;
providing a second articulation section adjacent to the first articulation section, the second articulation section having a distal or proximal edge with a first inner ear comprising a recessed rivet receiving portion having an inner rivet receiving hole and a second inner ear comprising a recessed rivet receiving portion having an inner rivet receiving hole and with a proximal or distal edge with a first outer ear comprising an outer rivet receiving portion having an outer rivet receiving hole and a second outer ear comprising an outer rivet receiving portion having an outer rivet receiving hole;
placing the first articulation section on cam assembly tooling loading pin, the loading pin having a cam surface configured to contact with a head of a first rivet and a head of a second rivet;
inserting a first rivet shaft of the first rivet into the inner rivet receiving hole of the first inner ear, wherein the first head of the first rivet is located at an interior side of the first inner ear and facing the cam surface;
inserting a second rivet shaft of the second rivet into the inner rivet receiving hole of the second inner ear, wherein the second head of the second rivet is located at an interior side of the second inner ear and facing the cam surface;
placing the second articulation section adjacent to the first articulation section with the inner rivet receiving hole of the first inner ear aligned with the outer rivet receiving hole of the first outer ear and with the inner rivet receiving hole of the second inner ear aligned with the outer rivet receiving hole of the second outer ear;
rotating the loading pin to press the cam surface of the loading pin against the first head of the first rivet to move the first rivet shaft into and partially through the outer rivet receiving hole of the first outer ear and to simultneously press the cam surface of the loading pin against the second head of the second rivet to move the second rivet shaft into and partially through the outer rivet receiving hole of the second outer ear;
fixedly connecting the first rivet to the second articulation section; and
fixedly connecting the second rivet to the second articulation section.

Fixedly connecting the first rivet to the second articulation section comprises welding the first rivet to the first outer ear of the second articulation section; and fixedly connecting the second rivet to the second articulation section comprises welding the second rivet to the second outer ear of the second articulation section.

In accordance with a further aspect of the invention, a method is presented, which entails the alignment of rivet holes in two endoscope articulation sections with each other. A rivet is inserted into these rivet holes, with one end (head) of the rivet positioned on the interior side of the first articulation section and the opposite end extending to the exterior side of the second articulation section. Instead of using a mandrel with a linear cam, a rotating cam is inserted into the articulation sections to align them and to contact the rivet head, thereby aligning the rivet relative to the articulation sections while maintaining the necessary clearance between the rivet head and the inner ring ear. Finally, the rivet is securely fixed to the second articulation section.

The various features of novelty which characterize the invention are pointed out with particularity in the claims annexed to and forming a part of this disclosure. For a better understanding of the invention, its operating advantages and specific objects attained by its uses, reference is made to the accompanying drawings and descriptive matter in which preferred embodiments of the invention are illustrated.

### BRIEF DESCRIPTION OF THE DRAWINGS

In the drawings:
Figure 1 is a perspective view of a flexible endoscope assembled with the method of assembling articulation sections according to this disclosure;
Figure 2 is a perspective view showing several joined articulation sections;
Figure 3 is a cross sectional view showing the joined articulation sections of Figure 2;
Figure 4 is a perspective view of an articulation section with two hinge points, in the form of upper and lower rivet receiving holes each defined by an inner ear (recess rivet receiving ring portion), and showing the articulation section on a cam assembly tooling loading pin;
Figure 5 is a perspective view of the arrangement shown in Figure 4, showing the insertion of a first rivet;
Figure 6 is a perspective view with an arrow that indicates a rotation of the arrangement shown in Figure 5;
Figure 7 is a perspective view of the arrangement shown in Figure 2 and showing insertion of a second rivet at an opposite side of the arrangement of the articulation section on the loading pin;
Figure 8 is a perspective view showing the positioning of further (other) articulation sections at a location adjacent to the first articulation section with the adjacent further articulation section shown as transparent to show a recessed rivet receiving hole of a recessed rivet receiving ring portion aligned with an outer rivet receiving hole of an outer rivet receiving ring portion of the adjacent further articulation section;
Figure 9 is a perspective view showing the positioned further articulation sections on a carrying mandrel to position the further articulation sections at a location adjacent to the first articulation section as shown in Figure 8;
Figure 10 is a cross sectional view showing the positioned further articulation sections on a carrying mandrel at a location adjacent to the first articulation section and with the first articulation section carried by a mandrel of the cam assembly tooling loading pin;
Figure 11 is a perspective view showing the rotation of the loading pin with a tooling support to effect a pressing of the rivet head to move the rivet shafts (rivet cylindrical portions) into and through the outer rivet receiving hole at each side;
Figure 12A is a cross-sectional view showing the cam assembly tooling loading pin in a first state prior to pressing the rivets; and
Figure 12B is a cross-sectional view showing the cam assembly tooling loading pin in a second state, rotated 90° relative to the first state, showing the rivet shafts pressed through each outer rivet receiving hole.

### DESCRIPTION OF THE PREFERRED EMBODIMENTS

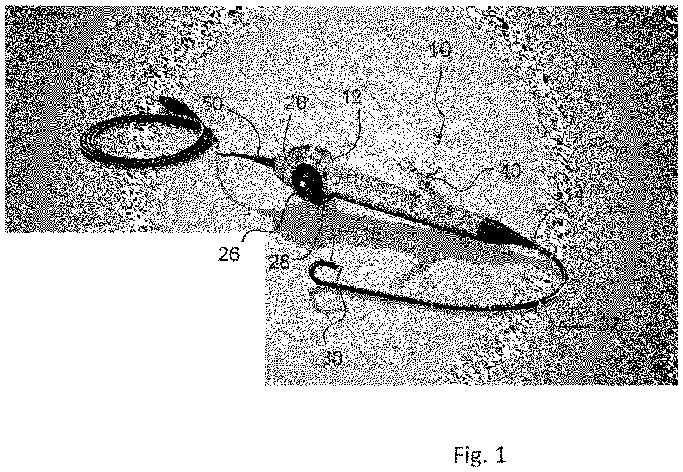

Referring to the drawings in particular, FIG. 1 shows a perspective view of an endoscope 10 assembled with the method of assembling articulation sections according to this disclosure. The endoscope 10 may be a ureteroscope or other suitable type of endoscope that includes articulated sections. The endoscope 10 generally comprises a handle or control 12 and a deflection section 16 that is located at a distal end of the handle 12. The endoscope 10 has a shaft 14 with the deflection section 16. A control system 20 to control the active deflection section 16 extends from the handle 12 to the deflection section 16. The control system 20 can comprise, for example, a pair of control wires, two wire sheaths, and an actuator 26. One end of the wires are connected to the actuator 26 and an opposite second end of the wires are connected to the distal end of the active deflection section 16.

In the preferred embodiment, the handle 12 has a user operated slide or lever 28. The lever 28 is connected to the actuator 26. The actuator 26 is adapted to pull and release the two wires of the control system 20. When the lever 28 is moved by the user, the actuator 26 is moved. The actuator 26 may be a drum or pulley, for example, rotatably connected to the handle 12 to pull one wire while releasing the other. The actuator may also be a rocker arm adapted to pull and release the wires of the control system 20. The control system 20 may also have two or more pairs of control wires. In this implementation the handle can have additional actuators and corresponding controls to drive the additional pairs of control wires to bend the deflection section in different plane(s). In further alternate embodiments, the handle 12 may have knobs with rack and pinion mechanisms or other suitable user operated controls for the control system.

The flexible shaft 14 with deflection section 16 includes the control wires of the control system 20, a fiber optical image bundle or video sensor electrical cable, a working channel, and a fiber optical illumination bundle or electrical wires to illumination LEDs or lights at an objective head 30. A port 40 for inserting accessory instruments (not shown) into the working channel is located on the handle 12. The handle 12 also has an electrical cable 50 for connection to another device, such as a video monitor for example. In an alternate embodiment, instead of the cable 50, the endoscope 10 could have an eyepiece. In alternate embodiments, the flexible shaft may house different systems therein.

The deflection section or steering/articulation section 16 generally comprises a frame that has a cover 32 and the objective head 30. The cover 32 also extends over the entire shaft 14, or over a portion or there may be different sections that have different cover portions. The frame has at least one portion comprising articulation sections 60 that are disposed adjacent to each other and are articulated to each other. The articulation sections 60 are comprised of stainless steel. The articulation sections 60 could be comprised of another material such as plastic or a shape memory alloy material, such as Tinel or Nitinol.

The articulation sections 60 each comprise a one-piece member, but could be comprised of multiple members. Each articulation section 60 has a first side connection edge 62, such as a proximal side connection edge, and a second side connection edges, such as a distal side connection edge 64. The first side connection edge 62 has an inner ear (recessed rivet receiving portion) 66 that defines a inner rivet receiving hole 68. The second side connection edge 64 has an outer ear (outer rivet receiving portion) 70 that defines an outer rivet receiving hole 72.

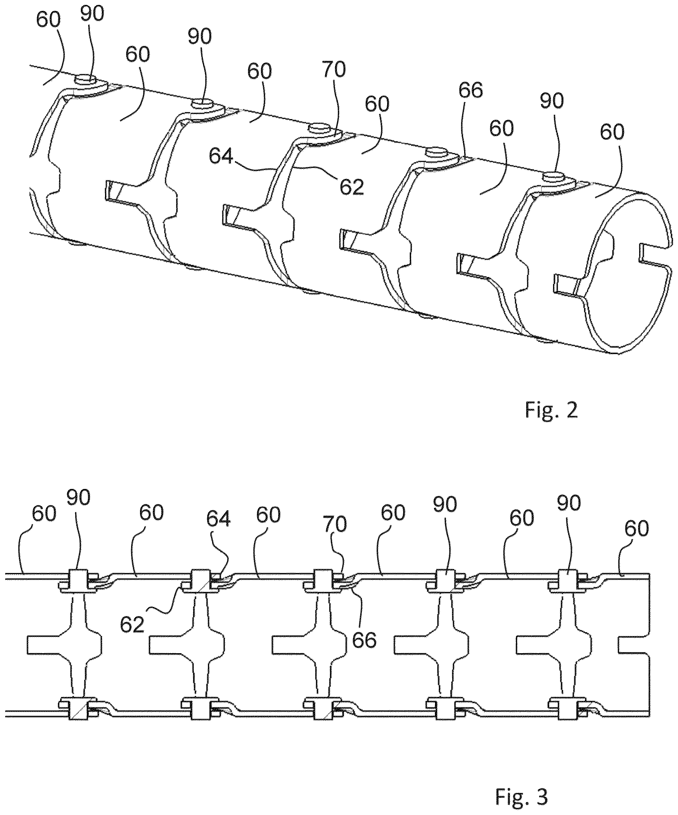

The articulation sections 60 are disposed adjacent to each other and are connected by rivets 90 so as to provide an articulated connection (Figures 2 and 3). The articulation sections 60 may have an identical configuration or this configuration may vary based on different size or other considerations. In the example of the method according to this disclosure essentially identical articulation section 60 are connected to each other based on principles of the method according to this disclosure.

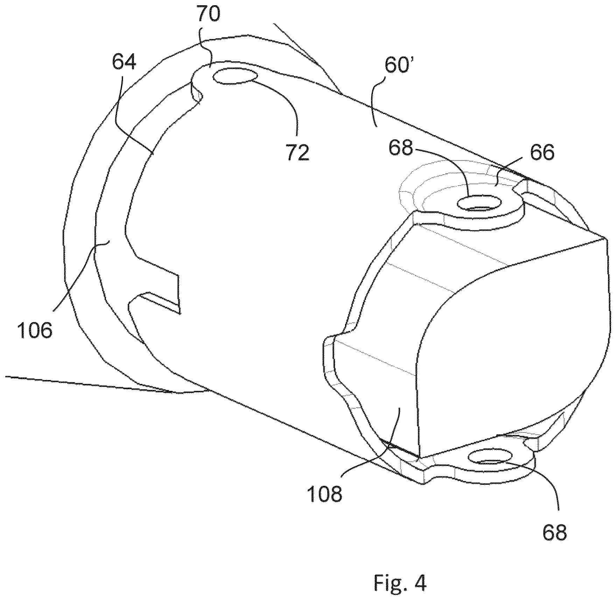

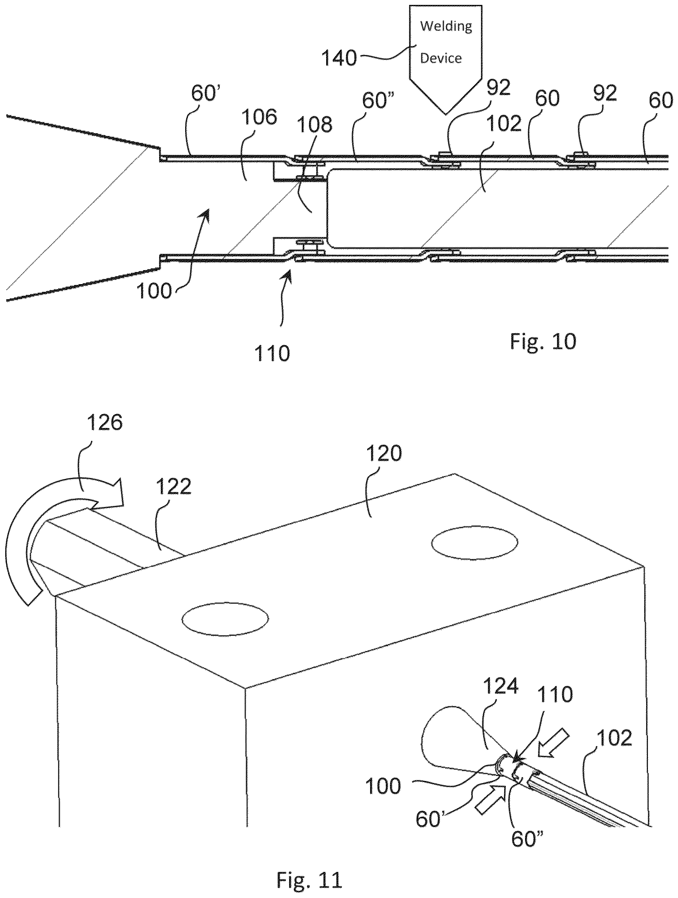

As can be seen in Figure 4 a next to be connected articulation section (first articulation section) 60', which is to be connected to a further adjacent articulation section (second articulation section) 60", is placed on a mandrel portion 106 of the cam assembly tooling mandrel and loading pin 100. The cam assembly tooling mandrel and loading pin 100 has the mandrel portion 106, for supporting the cylindrical inner surface of the next to be connected articulation section 60' and the loading pin portion 108, that has the cam surface 112 discussed further below. The next to be connected articulation section 60' is disposed with the first side connection edge 62 having the inner ear 66 at upper and lower locations, exposed for connection. The cam assembly tooling mandrel and loading pin 100 is shown with the to be connected articulation section 60 on the mandrel portion 106. As shown in Figure 11, the cam assembly tooling mandrel and loading pin 100 is supported by support block 120 with a support portion 124 and is rotated by actuator 122 as indicated by rotation arrow 126.

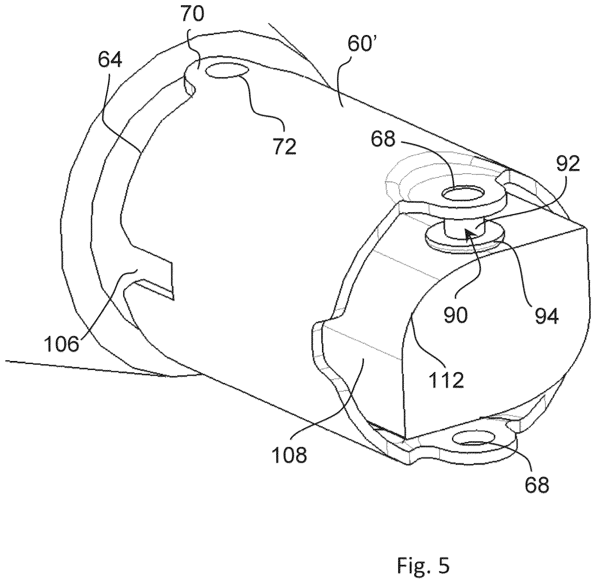

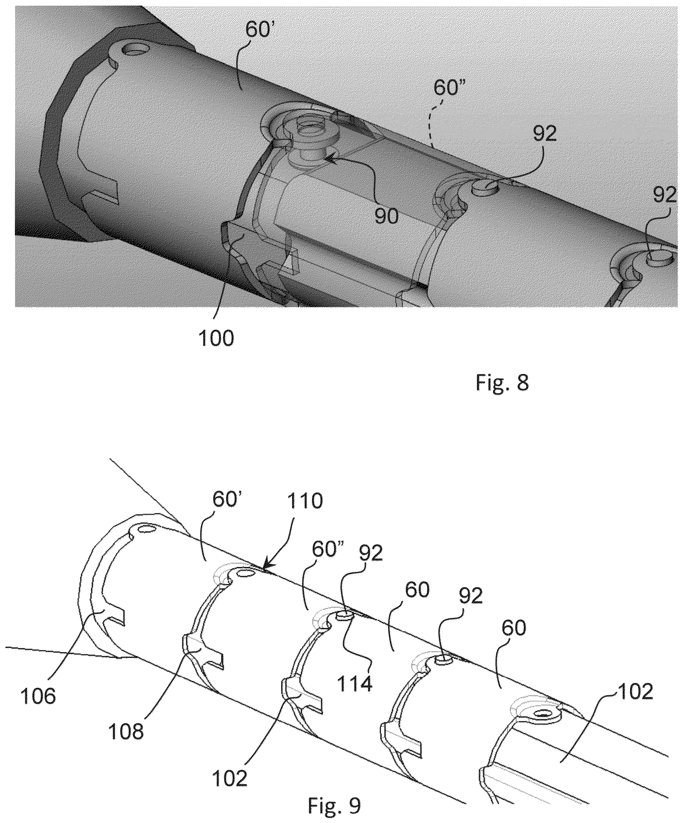

As can be seen in Figure 5, a rivet 90 is positioned with a rivet shaft (cylindrical body) 92 in the inner rivet receiving hole 68 of the next to be connected articulation section 60'. The positioned rivet head 94 of the rivet 90 is shown adjacent to and in contact with the cam surface 112 of the loading pin 108. Figures 8, 9 and 10 show further articulation sections 60. A left most further articulation sections 60" has the second side connection edge 64 facing the first side connection edge 62 of next to be connected articulation section 60'. The second side connection edge 64 has the outer rivet receiving hole 72 of the outer ear 70.

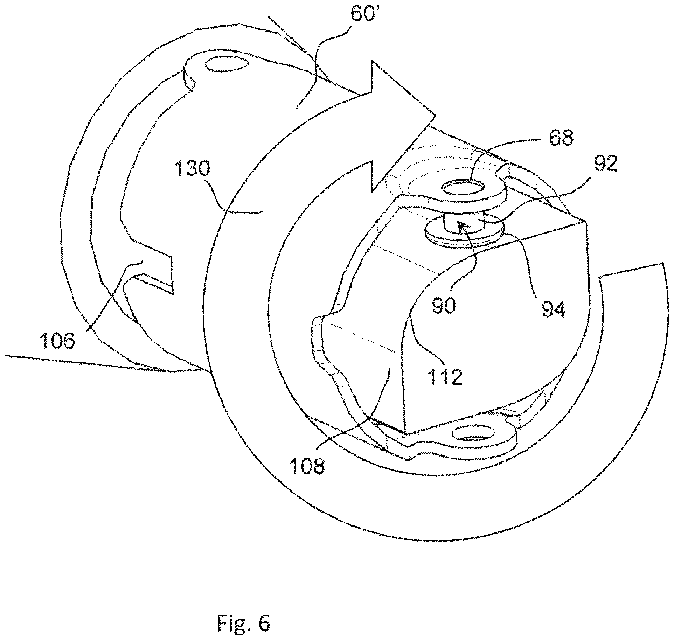

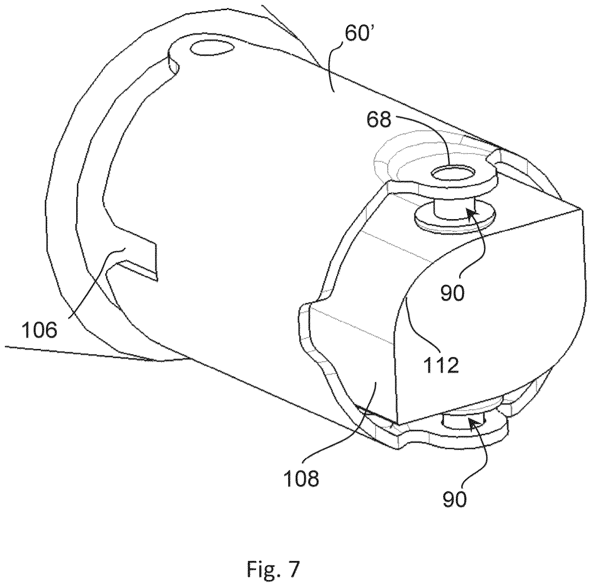

Figure 6 shows the same state of the arrangement of articulation section 60 to be connected on the cam assembly tooling loading pin 100 and indicates a step of rotating the articulation section 60' to be connected as shown by arrow 130. From the state shown in Fig. 6, the next to be connected articulation section 60' is rotated by 180° to position the next to be connected articulation section 60' in a position as shown in Figure 7. A rivet shaft 92 of another rivet 90 is inserted in another inner rivet receiving hole 68, at the opposite side of the first side connection edge 62. After the other rivet 90 is positioned as shown in Figure 7, two rivets are disposed in the respective inner rivet receiving holes 68 (one at top one at bottom) as can be seen in Figure 7. Although the method may include rotation of articulation section 60 to be connected with the assembly tooling mandrel and loading pin 100, this is not required as the second rivet may be simply inserted at the lower rivet receiving hole 68 without rotation of the arrangement of articulation section 60 to be connected on the cam assembly tooling mandrel and loading pin 100.

Figures 8 and 9 show the further step of placing the further articulation sections 60 at a location immediately adjacent to the next to be connected articulation section 60'. In Figure 8, the left most further articulation section or adjacent further articulation section 60" is shown as transparent. The further articulation sections 60 are shown with respective rivets in a pre-weld connected state. These further articulation sections 60 are carried by support mandrel 102 and positioned at the location with the left most (adjacent) further articulation section 60" immediately adjacent to the next to be connected articulation section 60'. This positions the adjacent further articulation section 60", carried on support mandrel 102, such that the outer rivet receiving hole 72 of the outer ear 70 is aligned with the inner rivet receiving hole 68 of the inner ear 66 of the nxt to be connected articulation section 60. This provides a pre-assembly 110 with the articulation section 60' to be connected supported by the mandrel portion 106 of the assembly tooling mandrel and loading pin 100 with the cam surface 112 facing and preferably contacting the two rivet heads 94, and with the adjacent (left most) further articulation section 60", supported by support mandrel 102.

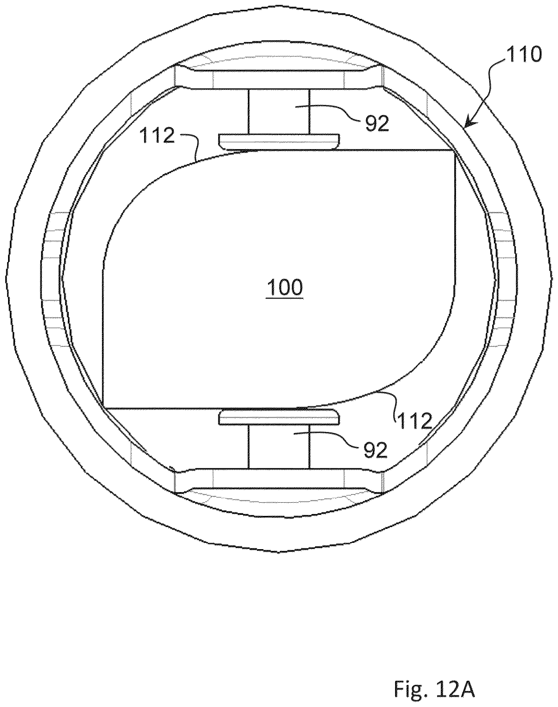

Figure 11 shows the pre-assembly 110, including the nect to be connected articulation section 60' and adjacent further articulation section 60" with aligned holes 698, 72 and with respective rivets 90 inserted only into the inner rivet receiving holes 68. This pre-assembly 110 is also shown in Figure 12A in cross-section. As indicated in Figure 11 the actuator 122 of support block 120 is rotated as indicated by rotation arrow 126. This rotates the assembly tooling mandrel and loading pin 100 such that the loading pin 108 with cam surface 112 rotates.

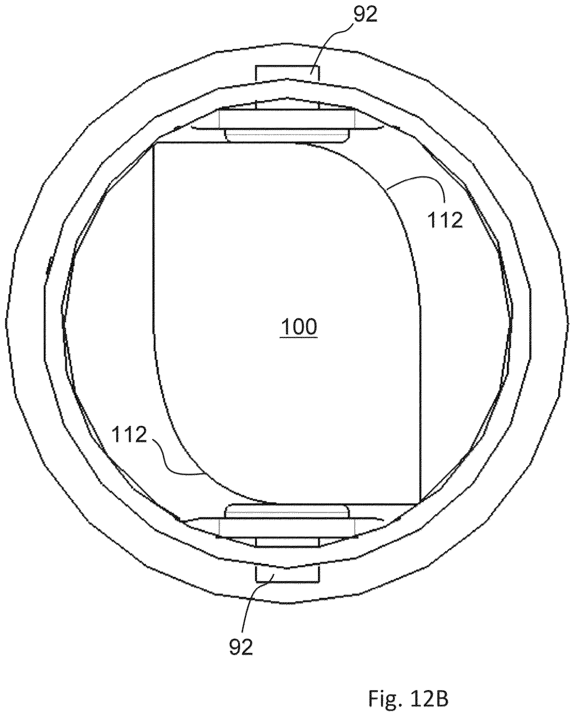

Figure 12B shows the loading pin 108 rotated, which results in the cam surface 112 pressing against the head 94 of the rivet 90 at each side. This rotation by 90° of the loading pin 108 results in each rivet shaft 92 being pressed through the inner rivet receiving hole 68 of the inner ear 66 and through the outer rivet receiving hole 72 of the outer ear 70. The cam surface 112 of the loaded pin 100 is pressed against the head 94 of each rivet 90 so as to position each rivet 90 in a pre-weld position at a known location with known clearance as shown in Figure 8B.

With each rivet 90 in its pre-weld position at the known location, each rivet 90 is fixedly connected to the adjacent further articulation section 60, in particular to the outer ear 70. Advantageously the fixing of each rivet 90 to the adjacent articulation section comprises welding the rivet to the further adjacent articulation section 60" at a region of the rivet shaft (cylindrical body) 92 adjacent to the outer ear 70. Preferably each articulated connection is brought to first a pre-weld state and subsequently each rivet shaft (cylindrical body) 92 of each rivet 90 is welded to a respective outer ear 70. This may be done with the rivets at one side of the chain of articulated connection in the pre-weld state being welded, one after another and subsequently rivets at the other side of the chain of articulated connection in the pre-weld state being welded, one after another.

The fixedly connected articulation sections 60 form a fixedly joined articulation section chain that comprises a plurality of such articulation sections 60, one after another. As noted, these articulation sections 60 may be of identical configuration but may also have varying axial extent. The first connection edges 62 of the articulation sections 60 may all be identical and the second connection edges 64 of the articulation sections 60 may be identical except for the distal last second side connection edge 64, which may have a different configuration (see Figures 2 and 3). Further, the first connection edge 62 of the chain of articulation section 60 may connect to a shaft section having a different configuration, namely without a second section connection edge 64 as this differently configured shaft section is not in the chain and acts to form the first articulated joint.

Although upper and lower rivets 90 are shown connecting each of the articulation sections 60 to be connected and adjacent further articulation section 60 after positioned with aligned holes 68 and 72, it may be possible to implement the flexible shaft at least partially with a single rivet. However, the invention advantageously allows plural rivets to be accurately positioned based on a single rotational movement of the cam assembly tooling loading pin 100. The disclosed method is particularly advantageous as to accurately positioning first and second rivets for welding without a need to linearly move the articulation sections 60 along with positioned rivets 90 along an assembly mandrel.

While specific embodiments of the invention have been shown and described in detail to illustrate the application of the principles of the invention, it will be understood that the invention may be embodied otherwise without departing from such principles.

### List of reference numbers:

- 10: Endoscope
- 12: handle/control
- 14: shaft
- 16: flexible / deflection section
- 20: control system 20
- 26: actuator
- 30: objective head
- 32: cover
- 50: electrical cable
- 60: articulation sections
- 60': next to be connected articulation section
- 60": adjacent further articulation sections
- 62: first side connection edge
- 64: second side connection edges
- 66: inner ear (recessed rivet receiving ring portion)
- 68: inner rivet receiving hole
- 70: outer ear (outer rivet receiving ring portion)
- 72: outer rivet receiving hole
- 90: rivet (articulation hinge point)
- 92: rivet shaft (cylindrical body)
- 94: rivet head
- 100: cam assembly tooling mandrel with loading pin having a cam surface
- 102: connected articulation sections support mandrel
- 110: preassembly of positioned first articulation section and second articulation section
- 112: cam surface
- 120: cam assembly tooling support block
- 122: actuator
- 124: cam assembly tooling mandrel widened portion
- 126: rotation of actuator to rotate loading pin relative to preassembly of positioned first articulation section and second articulation section
- 130: rotation loading pin and first articulation section assembly

## Claims

1. A method comprising:
providing a first articulation section (60') comprising a first section connection edge (62) with an inner ear (66) comprising a recessed rivet receiving portion having an inner rivet receiving hole (68) and having an opposite section edge;
providing a second articulation section (60") comprising a second section connection edge (64) with an outer car (70) comprising an outer rivet receiving portion having an outer rivet receiving hole (72) and having an opposite section edge;
placing the first articulation section on a cam assembly tooling mandrel and loading pin (100), the loading pin (108) having a cam surface (112) configured to contact with a head of a rivet (94);
inserting a rivet shaft (92) of the rivet (90) into the inner rivet receiving hole of the inner ear (68), wherein the head of the rivet (94) is located at an interior side of the inner ear (66) and facing the cam surface (112);
placing the second articulation section (60") adjacent to the first articulation section (60') with the outer rivet receiving hole (72) of the outer ear (70) aligned with the inner rivet receiving hole (68) of the inner ear (66);
rotating the loading pin (108) to press the cam surface (112) of the loading pin (108) against the head of the rivet (94) to move the rivet shaft (92) partially through inner rivet receiving hole (68) of the inner ear (66) and into and partially through the outer rivet receiving hole (72) of the outer ear; and
fixedly connecting the rivet to the second articulation section (60").

2. A method as in claim 1, wherein fixedly connecting the rivet (90) to the second articulation section (60") comprises welding the rivet to the outer ear (70) of the second articulation section (60").

3. A method as in claim 1, wherein:
the first section connection edge (62) of the first articulation section (60') further comprises another inner ear comprising another recessed rivet receiving portion having an another inner rivet receiving hole;
the second section connection edge of the second articulation section (60") further comprises another outer ear (70) comprising another outer rivet receiving portion having another outer rivet receiving hole; and
another rivet is provided.

4. A method as in claim 3, further comprising inserting another rivet shaft of the other rivet into the other inner rivet receiving hole (68) of the other inner ear (66), wherein another head of the other rivet (94) is located at an interior side of the inner ear and facing the cam surface (112), wherein the cam surface (112) is configured to contact with the head of the rivet (94) and also to contact a head of the other rivet (94), whereby the rotation of the loading pin (108) presses the cam surface (112) of the loading pin (108) against both the head of the rivet (94) to move the rivet shaft into and partially through the outer rivet receiving hole (72) of the outer ear (70) and also the head of the other rivet (94) to move the other rivet shaft (92) into and partially through the other outer rivet receiving hole (72) of the other outer ear.

5. A method comprising:
providing a first articulation section (60') having a section distal or proximal edge with an first inner ear comprising a recessed rivet receiving portion having an inner rivet receiving hole (68) and an second inner ear (66) comprising a recessed rivet receiving portion having an inner rivet receiving hole (68) and with a proximal or distal edge with a first outer ear (70) comprising an outer rivet receiving portion having an outer rivet receiving hole (72) and second outer ear (70) comprising an outer rivet receiving portion having an outer rivet receiving hole (72);
providing a second articulation section (60") adjacent to the first articulation section, the second articulation section (60") having a distal or proximal edge with a first inner ear comprising a recessed rivet receiving portion having an inner rivet receiving hole (68) and a second inner ear (66) comprising a recessed rivet receiving portion having an inner rivet receiving hole (68) and with a proximal or distal edge with a first outer ear (70) comprising an outer rivet receiving portion having an outer rivet receiving hole (72) and a second outer ear (70) comprising an outer rivet receiving portion having an outer rivet receiving hole (72);
placing the first articulation section (60') on cam assembly tooling loading pin, the loading pin (108) having a cam surface (112) configured to contact with a head of a first rivet (94) and a head of a second rivet (94);
inserting a first rivet shaft (92) of the first rivet (90) into the inner rivet receiving hole (68) of the first inner ear (66), wherein the first head of the first rivet (94) is located at an interior side of the first inner ear and facing the cam surface (112);
inserting a second rivet shaft (92) of the second rivet (90) into the inner rivet receiving hole (68) of the second inner ear (66), wherein the second head of the second rivet (94) is located at an interior side of the second inner ear and facing the cam surface (112);
placing the second articulation section adjacent to the first articulation section (60') with the inner rivet receiving hole (68) of the first inner ear (66) aligned with the outer rivet receiving hole (72) of the first outer ear (70) and with the inner rivet receiving hole of the second inner ear (68) aligned with the outer rivet receiving hole (72) of the second outer ear;
rotating the loading pin (108) to press the cam surface (112) of the loading pin (108) against the first head of the first rivet (94) to move the first rivet shaft (92) into and partially through the outer rivet receiving hole (72) of the first outer ear (70) and to simultaneously press the cam surface (112) of the loading pin (108) against the second head of the second rivet (94) to move the second rivet shaft (94) into and partially through the outer rivet receiving hole (72) of the second outer ear (70);
fixedly connecting the first rivet to the second articulation section; and
fixedly connecting the second rivet to the second articulation section.

6. A method as in claim 5, wherein:
fixedly connecting the first rivet (90) to the second articulation section (60") comprises welding the first rivet to the first outer ear (70) of the second articulation section (60"); and
fixedly connecting the second rivet to the second articulation section (60") comprises welding the second rivet to the second outer ear of the second articulation section.
